# EUROPEAN PATENT APPLICATION

(11) **EP 3 988 047 A1**
(43) Date of publication of application: **27.04.2022**
(21) Application number: 21150676.1
(22) Date of filing: 08.01.2021
(51) Int. Cl.: A61B 90/94, A61B 90/98, A61F 17/00, A61J 1/00, A61B 90/92

(54) **SYSTEM FOR MONITORING THE EXPIRY DATE**

(30) Priority: 26.10.2020 IT 202000025306
(71) Applicant: H24INVENT SRL, 30172 Mestre, Venezia (IT)
(72) Inventor: Ranzato, Enrico, 30172 Venezia (IT)
(74) Representative: Leganza, Alessandro

(57) **Abstract**

The present invention relates, in general, to a system for monitoring the expiry date of drugs or first aid or emergency kits and, more particularly, to a system configured to inform a user about the expiration of drugs.

## Description

### Tecnhical field

The present invention relates, in general, to a system for monitoring the expiry date of drugs or first aid or emergency kits and, particularly, to a system configured to inform a user about the expiration of drugs

### Background of the invention

Drugs or pharmaceutical products consist of compositions comprising one or more pharmaceutically active substances and one or more pharmaceutically allowable excipients. These drugs tend to undergo different types of degradation, for example thermal, photochemical hydrolysis, etc. therefore, after a certain period they expire, that is they can no longer be consumed because the original composition has been modified over time in such a way that their consumption is no longer safe.

It is estimated that, nowadays, a large part of the sold drugs are lost due to deterioration.

To protect the health of users, all drugs are provided with an expiry date.

The expiry date is typically affixed to the drug packaging and indicates the date by which a drug is suitable for being taken, whether kept in the correct storage conditions.

Typically, the expiry date consists of the indication, in the order of the day, of month and possibly of the year. Once the expiry date has passed, the drug can constitute a health hazard due to the degradation products that are generated, and therefore it can no longer be consumed.

In modern society, life is becoming more and more chaotic and often consumers, once they have bought one or more drugs, and, despite the expiry date reported on the packagings, they forget to take them by that date.

Furthermore, a further problem is that particularly felt by visually impaired users as such subjects, having difficulty to quickly view the expiry dates, would need systems that warn them about the approaching expiration of drugs or warn them whether the drug has expired.

In addition to drugs, also first aid or emergency kits, which contain drugs and/or medication devices, are subject to expiration.

Such kits are often stored in a first aid or emergency box, bag, kit (30) which is placed in proximity to potentially dangerous situations, for example, close to laboratories, in cars, in factories, but as well in offices. Since these kits are indeed used in emergency situations, their use is occasional or rare, or very rare and it may happen that, due to inattention, the kit has expired exactly when its use is absolutely necessary in situations of accident and/or where people's lives are at stake. Therefore, it is of extraordinary importance to monitor the expiry date of first aid or emergency kits, thus to replace them nearby their expiry date with newer kits.

### Summary of the invention

Therefore, the problem addressed by the present invention is that of providing a system for monitoring the expiry date of a drug or a first aid or emergency kit, in order to overcome, or at least to limit, the drawbacks mentioned above.

In particular, the main object of the present invention is to provide a system for monitoring the expiry date of a drug or of a first aid or emergency kit with the function of indicating to the consumer the imminent expiration of a drug and such that it can operate in any storage environment of the drug or kit, i.e. both in low temperature environments, for example inside a refrigerator, and in environments without light, for example, inside a medicine cabinet.

Another object of the present invention is that to provide a system for monitoring the expiry date of a drug or a first aid or emergency kit with the function of indicating to the consumer he imminent expiration, which has a simplified structur and low production costs.

Not the least object of the present invention is to provide a system for monitoring the expiry date of a drug or a first aid or emergency kit, with the function of indicating to the consumer the imminent expiration of the same, which is easy and immediate its use.

An object of the invention relates, therefore, to a system (100) for monitoring the expiry date of a drug or a first aid or emergency kit comprising:
- at least one intelligent portable device (50),
- a drug packaging (20) containing a drug or a first aid or emergency box, bag or kit (30) containing drugs and/or medication devices, wherein said packaging (20) or box, bag or kit (30) comprises at least one wireless communication module (40) configured to communicate with a corresponding wireless communication module (51) of an intelligent portable device (50).

It is evident how the presence of the wireless communication module (40) allows to inform a user about the expiration of the drug or the first aid or emergency kit.

Another object, is a method for monitoring the expiration of a drug or first aid or emergency kit.

Finally, another object is a method for monitoring the expiration of a drug or a first aid or emergency kit by a visually impaired user.

Further features and advantages of the system and methods for monitoring the expiration of a drug or a first aid or emergency kit will become clearer from the detailed description, from the description of preferred embodiments, of the embodiment examples of the invention, provided as an indication of the invention.

Preferred aspects of the invention are reported in the dependent claims.

### Brief description of the drawings

With reference to the annexed drawings, the following elements are considered:
(100) system for monitoring the expiration of a drug or a first aid or emergency kit,
(90) first aid or emergency box, bag or kit (30) including at least one wireless communication module (40),
(91) drug packaging (20) containing a drug comprising at least one wireless communication module (40),
(20) drug packaging (20) containing a drug,
(30) first aid or emergency box, bag or kit,
(40) wireless communication module,
(50) intelligent portable device,
(51) wireless communication module of an intelligent portable device,
(41) a memory unit,
(70) visual signalling means,
(42) timer,
(71) LED,
(72) embossing of an alphanumeric or Braille character,
(53) sound communication means,
(52) application of intelligent portable device,
(60) wireless communication module (40) of the portable type,
(61) hooking tab of (60) to a drug packaging.

Figure 1 is a perspective view of a first embodiment, wherein the part (90) of the system (100) for monitoring the expiry date of a first aid or emergency kit object of the present invention comprising:
(30) first aid or emergency box, bag or kit, (40) wireless communication module, (41) a memory unit inside the wireless communication module (40), (70) visual signalling means, (42) timer, (71) LED.

Figure 2 is a perspective view of a first embodiment, wherein the part (91) of the system (100) for monitoring the expiration of a drug object of the present invention comprising:
(20) drug packaging (20) containing a drug, (40) wireless communication module, (41) a memory unit inside the wireless communication module (40), (70) visual signalling means, (42) timer, (71) LED.

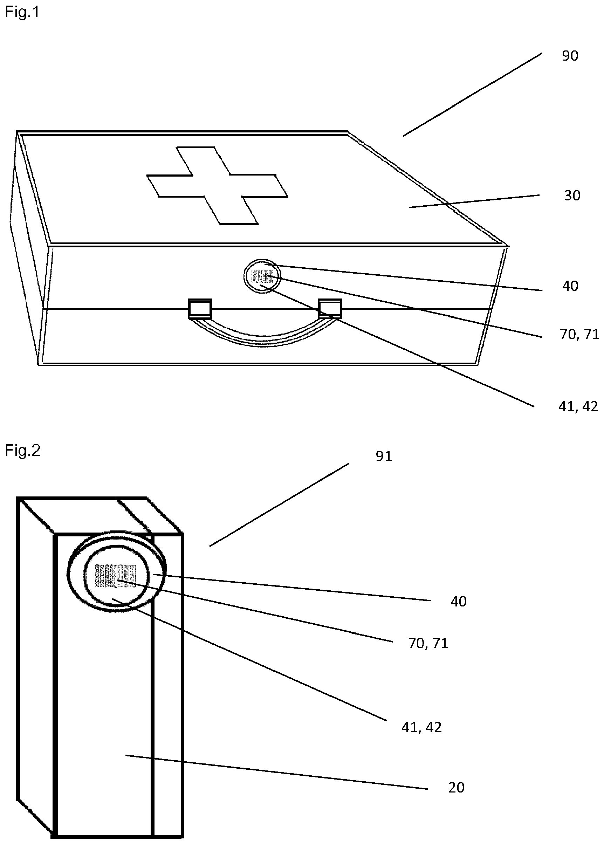

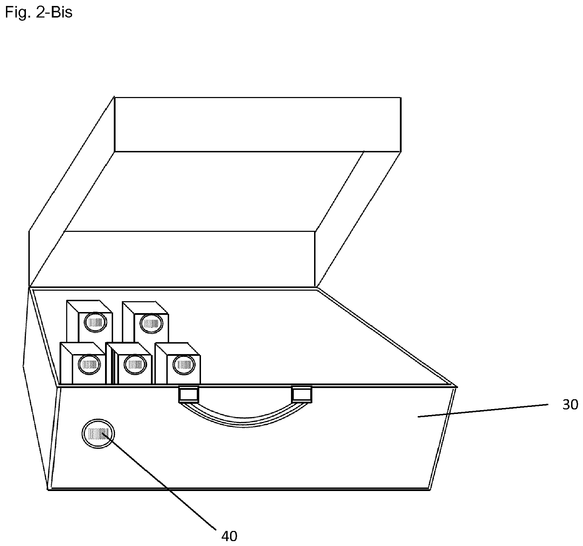

Figure 2-bis shows the aforementioned part (90) and the aforementioned part (91) of the system (100) for monitoring the expiration respectively of a first aid or emergency kit or the expiration of a drug.

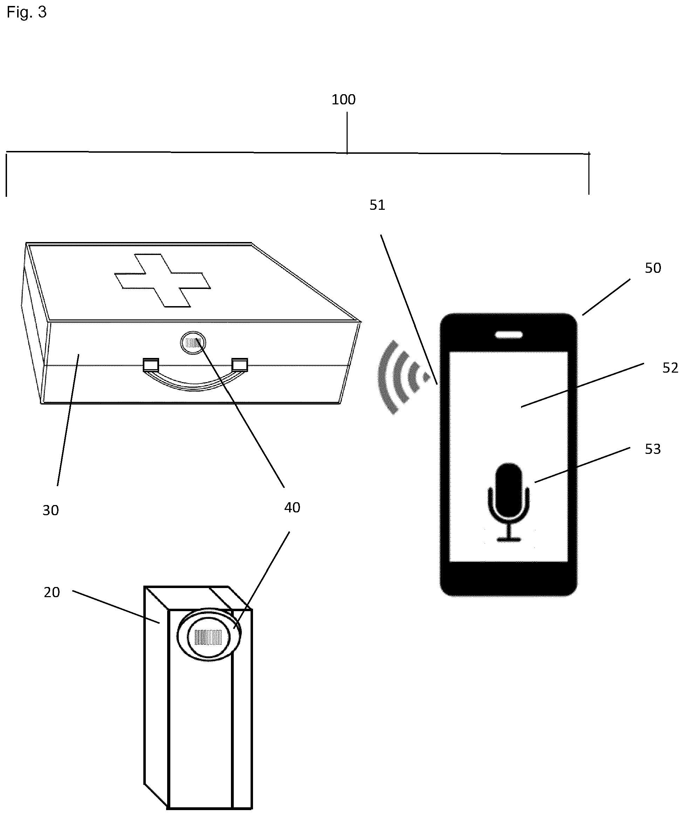

FIG. 3 is a perspective view of the entire system (100) for monitoring the expiration of a first aid or emergency kit (higher part) or drug (lower part) object of the present invention.

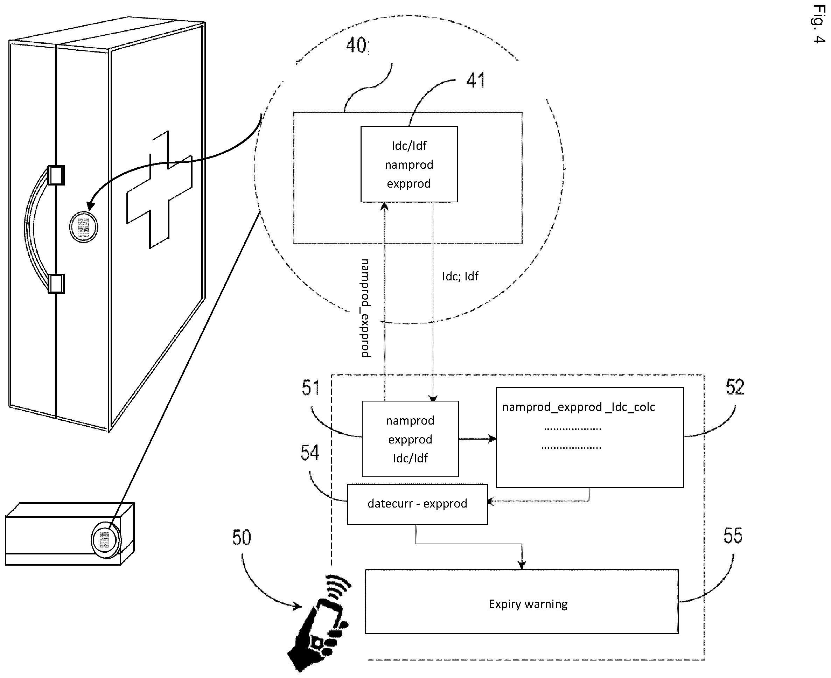

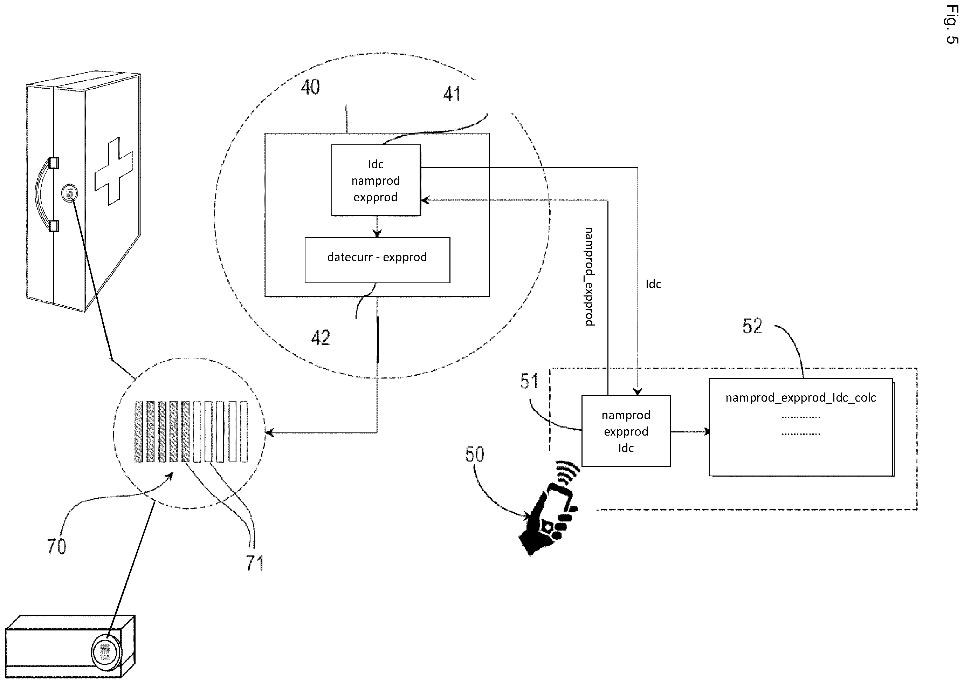

Figures 4 and 5 are diagrams that schematically show alternative operating modes of the system (100) object of the present invention. In particular, in Fig. 4 the approaching expiry date is indicated by means of the application of the intelligent portable device (52), while in Fig. 5 the approaching expiry date is indicated by means of visual signalling means (70).

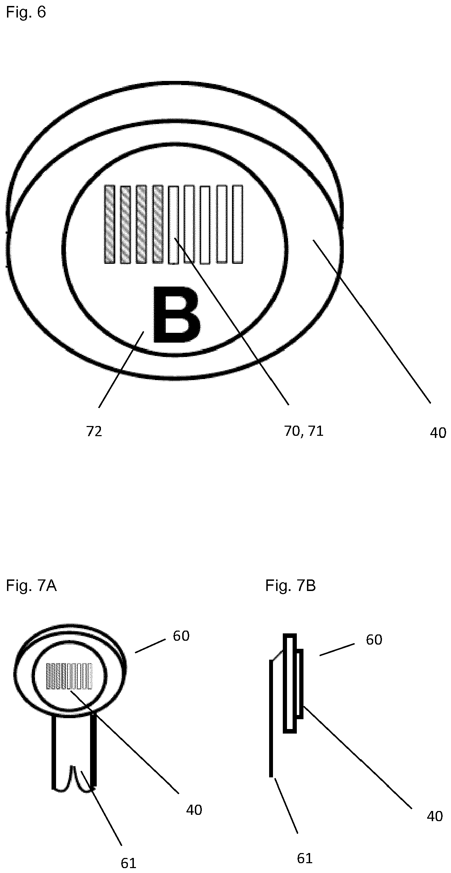

Fig. 6 shows a wireless communication module (40) having an embossing of an alphanumeric or Braille character (72), in the specific case the letter "B" is embossed.

Fig. 7A shows a perspective image of the wireless communication module (40) of the portable type (60). Look at the front part of the tab (61), below.

Fig. 7B shows a side image of the wireless communication module (40) of the portable type (60). Look at the side part of the tab (61).

### Detailed description of the invention and preferred embodiments

With reference to Figures from 1 to 3, these show a first embodiment of the system (100) for monitoring the expiry date of a drug or a first aid or emergency kit according to the present invention.

In particular, an object of the present invention is a system (100) for monitoring the expiry date of a drug or a first aid or emergency kit comprising:
- at least one intelligent portable device (50),
- a drug packaging (20) containing a drug or a first aid or emergency box, bag or kit (30) containing drugs and/or medication devices, wherein said packaging (20) or box, bag or kit (30) comprises at least one wireless communication module (40) configured to communicate with a corresponding wireless communication module (51) of an intelligent portable device (50).

It has been indeed surprisingly found that said combination of elements and means allows to effectively monitor the expiration of a drug or a first aid or emergency kit.

The first aid or emergency box, bag or kit (30) containing drugs and/or medication devices is also generically called first aid or emergency kit.

In the following description, and in the annexed claims, the expression "intelligent portable device (50)" is understood to refer to any type of electronic portable device able to be connected to other devices or to a network by means of a communication protocol, such as Bluetooth^{®}, NFC, Wi-Fi, 3G, etc., which can operate in an interactive and autonomous manner. Examples of such intelligent portable devices include a smartphone, an iPhone^{®}, a palmtop computer or PDA (Personal Digital Assistant), a tablet, a laptop, and similar devices.

In the following description, and in the annexed claims, the expression "drug" is understood to refer to a drug contained in a drug packaging (20).

The drug packaging (20) can be for example a box, a container, or a blister.

The drug packaging (20) can be made of for example of cardboard, carton and/or plastic material and/or at least partially of metallic material.

The first aid or emergency box, bag or kit (30) containing drugs and/or medication devices can be rigid or soft; it can be made of several materials, for example, of fabric, carton, soft or rigid plastic, metal, etc.

The system (100) has a wireless communication module (40) linked or rather attached to a drug packaging (20) or to the first aid or emergency box, bag or kit (30). This coupling can take place by means of mechanical fixing means and/or by welding or gluing, or, alternatively, by means of a magnet system.

Preferably, the wireless communication module (40) is a short-range wireless communication module, for example a Bluetooth^{®} wireless communication module or an NFC (Near Field Communication) wireless communication module.

The wireless communication module (40) is configured to communicate with an intelligent potable device (50) of the user (see Figures 3, 4 and 5).

Preferably, and as shown schematically in Figures 4 and 5, the wireless communication module (40) comprises a memory unit (41) configured to store data relating to the drug or the first aid or emergency kit, specifically the name of the drug or kit and its expiry date, transmitted to the wireless communication module (40) by the intelligent portable device (50).

For this purpose, and as shown in detail in Figures 4 and 5, the intelligent portable device (50) comprises a wireless communication module (51), preferably analogous to the wireless communication module (40) of the drug packaging (20) or a first aid or emergency box, bag or kit (30), and an application (52). Inside the memory unit (41) of the wireless communication module (40) is moreover stored an identification code of the first aid or emergency box, bag or kit (30) comprising at least one wireless communication module (40) (as a whole (90)) or of the drug packaging (20) comprising at least one wireless communication module (40) (as a whole (91)).

In an alternative embodiment, shown in Figure 5, the wireless communication module (40) further comprises visual signalling means (70), configured to indicate to the user how many days are left before the expiration of a drug or a first aid or emergency kit linked to at least one wireless communication module (40) (as a whole respectively (91) and (90)).

In particular, the visual signalling means (70) are provided on the outer surface of the wireless communication module (40) opposite to that of the outer surface of the drug packaging (20) or of a first aid or emergency kit (30). Preferably, the visual signalling means (70) are provided at the centre of the outer surface of the wireless communication module (40) opposite to that of the external surface of the drug packaging (20) or of a first aid or emergency kit. (30), so as to be easily and immediately visible by the user. Certainly, the visual signalling means can be positioned at any other point of the wireless communication module (40), provided they satisfy the condition of being visible to the user.

In the embodiment shown in Figure 1, 2, 3, 5, 6 and 7 the visual signalling means (70) comprise an array of LEDs (Light Emitting Diodes) 71, for example ten LEDs, one for each day left before to the expiration.

In this variant embodiment, the wireless communication module (40) is an wireless communication module of the active type, preferably an active NFC module, which comprises a timer (42).

The timer (42) is configured to perform a countdown between a current date datecurr and expiry date expprod of the drug or first aid or emergency kit linked to the wireless communication module (40), and, as to this approaching expiry date expprod, to activate the lighting-up of a number of LEDs (71) equal to the number of days left before the expiration of the drug or kit.

The wireless communication module (40) further comprises battery power supply means of known type (not shown) for the LEDs 71 and the timer 42.

With reference to Figure 4, the operation of the system (100) for monitoring the expiry date of drugs according to the present invention is now described.

In the following of the present description reference will be made, by way of intelligent portable device (50), to a smartphone or I-Phone^{®} (50), but it is understood that the comments are also applicable to any intelligent portable device available to the user and suitable for purpose.

Firstly, the user, by means of the application (52) being present inside the smartphone (50), enters the name namprod and expiry date expprod of a drug or a first aid or emergency kit. For example <aspirin; 1_october_2020>.

Subsequently, the user moves the smartphone (50) towards the wireless communication module (40) of the drug packing (20) or of the first aid or emergency box, bag or kit (30), typically at a distance not more than 10 cm, where the smartphone (50) is able to detect the presence of the wireless communication module (40).

At this point, the wireless communication module (40) and the wireless communication module (51) of the smartphone (50) are able to communicate with each other.

In particular:
- the wireless communication module (40) transmits to the wireless communication module (51) of the smartphone (50) the identification code Idc of said communication module (40), which is stored in the memory (41); and
- the wireless communication module (51) of the smartphone (50) transmits to the wireless communication module (40) the name namprod and expiry date expprod of the previous drug or kit entered by the user through the application (52) of the smartphone (50), for example <aspirin; 1_october_2020>.

These data are stored, together with the identification code Idc, in the memory (41) of the wireless communication module (40).

Following this exchange of information, there is an association between drug or between first aid or emergency box, bag or kit, expiry date of the drug or kit and wireless communication module (40). This association, for example <aspirin; 1_ottobre_2020_ldc> or <antibiotic-29_ottobre_2020_ldf> is present both in the memory (41) of its wireless communication module (40) and in the application (52) of the smartphone (50).

Preferably, a colour is associated with the wireless communication module (40). More preferably, the wireless communication module (40) is coloured. It follows that, by means of the application (52), the user can associate with a drug or a first aid or emergency kit, expiry date, identification code Idc or Idf wireless communication module (40) as well the corresponding colc or coif color, for example, in the case of the wireless communication module (40) <aspirin; 1_october_2020_ldc_red>.

A completely similar procedure can be followed for any wireless communication module (40).

The smartphone (50) includes a timer (54), by means of which the application (52) can perform a countdown between the current date datecurr and the expiry date expprod of each drug or kit and, close to this expiry date expprod, for example when there are two days left before the expiration of the product, i.e. expprod - datecurr = 2, display on the screen (55) of the smartphone (50), for each product, an "imminent expiry" warning.

The user is thus informed that, for example, the drug or kit relating to the communication module (40) of red clour is expiring and therefore must be consumed or replaced.

Assume that, alternatively, the user wishes to know the expiry date of one or more drugs contained, for example, in his/her refrigerator.

For this purpose, the user moves the smartphone (50) towards the wireless communication modules (40) linked to the drugs of interest. The wireless communication module (40) then transmits to the wireless communication module (51) of the smartphone (50) the name namprod and expiry date expprod of the drug, and this information is displayed, by means of the application (52), on the screen (55) of the intelligent portable device (50) in the form of an "imminent expiry" warning.

In the case of first aid or emergency kits, once the first aid or emergency kit contained inside the box or bag (30) has expired or replaced, the wireless communication module (40) can be reused by resetting.

To this end, the memory (41) of the wireless communication module (40) can be deleted and it is possible to store on it, according to the method above described, name and expiry date of a new first aid or emergency kit.

With reference to Figure 5, the operation of the system (100) is now described, in its embodiment comprising a wireless communication module (40) of the active type, a timer (42) and visual signalling means (70).

As well in this case, as described above with reference to Figure 4, firstly the user, by means of the application (52) being inside the smartphone (50), enters the name namprod and expiry date expprod of an drug or kit linked to the wireless communication module (40).

Subsequently, the user moves the smartphone (50) towards the wireless communication module (40), typically at a distance not more than 10 cm, where the smartphone (50) is able to detect the presence of the wireless communication module (40).

At this point, the wireless communication module (40) and the wireless communication module (51) of the smartphone (50) are able to communicate with each other.

In particular:
- the wireless communication module (40) transmits to the wireless communication module (51) of the smartphone (50) an identification code Idc of the wireless communication module (40); and
- the wireless communication module (51) of the smartphone (50) transmits to the wireless communication module (40) the name namprod and expiry date expprod of the drug or kit, previously entered by the user. These data are stored, together with the identification code Idc, in the memory (41) of the wireless communication module (40).

Following this exchange of information, there is an association between drug or kit relating to wireless communication module (40), expiry date of drug or kit and wireless communication module (40).

As described previously, a colour is preferably associated with the wireless communication module (40). It follows that, by means of the application (52), the user can associate with a drug or kit, expiry date, Idc identification code of the wireless communication module (40), also the related colc colour.

At this point, assume that the user wishes to know wheter the drug or kit linked to the wireless communication module (40) is expiring.

For this purpose, the user moves the smartphone (50) towards wireless communication module (40), in order to activate the wireless communication module (40), in particular the timer (42). Once activated, the timer performs a countdown between the current date datecurr and the expiry date expprod of the drug or the kit and activates the lighting-up of a number of LEDs, for example five LEDs, equal to the number of days left before the expiration. The user is then informed that the drug or kit will expire within five days.

According to a preferred embodiment, the system (100) comprises an identification code (Idc; Idcon), said wireless communication module (40) being configured to transmit to the intelligent portable device (50) said identification code (Idc; Idcon).

According to a preferred embodiment, the wireless communication module (40) comprises a memory unit (41), and wherein the wireless communication module (51) of the intelligent portable device (50) being configured to transmit to the wireless communication module (40) name (namprod) and expiry date (expprod) of a specific drug or kit, wherein said name (namprod) and expiry date (expprod) were previously entered into the intelligent portable device (50) by means of an application (52) of the intelligent portable device (50), the name (namprod) and the expiry date (expprod) being stored in said memory unit (41).

According to a preferred embodiment, the wireless communication module (40) of the system (100) is further configured to transmit to the intelligent portable device (50) said name (namprod) and expiry date (expprod), therein previously stored in the memory unit (41).

According to a preferred embodiment, the system (100) further comprising visual signalling means (70) and a timer (42) in electrical communication with said visual signalling means (70), said timer (42) being configured to perform a countdown between a current date (datecurr) and the expiry date of the drug or kit (expprod) and activate lighting-up of the visual signalling means (70).

According to a preferred embodiment, the aforementioned visual signalling means (70) comprise an array of LEDs (71) and wherein said timer (42) is configured to activate the lighting-up of a number of LEDs (71) equal to the difference between the expiy date of the drug or kit (expprod) and the current date (datecurr).

According to a preferred embodiment of the system (100),
- the wireless communication module (40) comprises an embossing of an alphanumeric or Braille character (72) on its outer surface opposite to that of the drug packaging (20) or of the first aid or emergency box, bag or kit (30), and
- the intelligent portable device (50) further comprises sound communication means (53). This preferred embodiment is indeed very useful for visually impaired users, allowing them to use the system (100) more effectively.

Alphanumeric or Braille character (72) means an alphabetic letter or a number or a letter or a number written in Braille writing.

Sound communication means (53) means that the intelligent portable device (50) has vocal commands or vocal or sound notifications, for example through a loudspeaker or headphones.

According to a preferred embodiment, the alphanumeric or Braille character (72) is an alphabetic letter, preferably capital letter, which is the initial of the colour of the wireless communication module (40).

Another object is a method for monitoring the expiry date of a drug or a first aid or emergency kit by the system described above, including any preferred embodiment, comprising the following steps:
a) providing a drug packaging (20) containing a drug or a first aid or emergency box, bag or kit (30) containing drugs and/or medication devices, wherein said packaging (20) or box (30) compraises at least one wireless communication module (40),
b) by means of the application (52) of the intelligent portable device (50), entering name (namprod) and expiry date (expprod) of a specific drug or first aid or emergency kit,
c) the intelligent portable device (50) transmits the data of step b) to the wireless communication module (40) which stores them in the memory unit (41),
d) the wireless communication module (40) by means of visual signalling means (70) and a timer (42), displays the residual duration time of the drug or of the first aid or emergency kit; or, alternatively, upon activation of the application (52) of the intelligent portable device (50), the intelligent portable device (50) warns the user of the approaching expiration of the drug or the first aid or emergency kit.

Steps a) and b) can also be reversed.

Another object is a method for visually impaired users for monitoring the expiry date of a drug or a first aid or emergency kit by the system (100) described above, including preferred embodiments, comprising the following steps:
a1) providing a drug packaging (20) containing a drug or a first aid or emergency box, bag or kit (30) containing drugs and/or medication devices, wherein said packaging (20) or box, bag or kit (30) comprises at least one wireless communication module (40), wherein said wireless communication module (40) comprises an embossing of an alphanumeric or Braille character (72) on its outer surface opposite to that of the drug packaging (20) or of a first aid or emergency box bag or kit (30),
b1) by means of the application (52) of the intelligent portable device (50), entering the name (namprod) and expiry date (expprod) of a specific drug or first aid or emergency kit,
c1) the intelligent portable device (50) transmits the data of step b1) to the wireless communication module (40) comprising an embossing of an alphanumeric or Braille character (72) which stores said data in the memory unit (41),
d1) optionally, the wireless communication module (40) by means of visual signalling means (70) and a timer (42), displays the residual duration time of the drug or the first aid or emergency kit,
e1) by means of the activation of the application (52) of the intelligent portable device (50), the intelligent portable device (50) by means of sound communication means (53) warns the visually impaired user of the approaching expiration of the drug or the first aid or emergency kit, moreover specifying the alphanumeric or Braille character of the wireless communication module (40),
f1) the user, through finger contact on the embossing of the alphanumeric or Braille character (72) of the communication module (40), recognizes the drug or the first aid or emergency kit close to the expiration.

Steps a1) and b1) can also be reversed.

It is evident that said method is particularly advantageous for visually impaired subjects, since it allows them to effectively monitor their expiry dates of own drugs or own first aid or emergency kits.

Preferably, in step a1) the embossing of a alphanumeric or Braille character (72) is one letter, preferably capital letter, which is the initial of the colour of the wireless communication module (40); for example, a white communication module (40) will have the embossing of the letter "B" on the wireless communication module (40).

According to an alternative embodiment, the wireless communication module (40) can be portable type. In particular, the wireless communication module (40) of the portable type (60) includes: a wireless communication module (40) and a tab (61).

The tab (61) is made in a shape such as to allow the hooking of the module (60) to the drug packaging.

Therefore, a further object is the use of a wireless communication module (40) of the portable type (60) configured to communicate with a corresponding wireless communication module (51) of an intelligent portable device (50) to monitor the expiry date of a drug or a first aid or emergency kit.

It is understood that wireless communication module (40) belonging to the portable type (60) has the same characteristics and constituent elements described above for the non-portable version and this also applies to the use of said communication module (40).

From the above-mentioned description the features of the system for monitoring the expiry date of drugs or of first aid or emergency kits object of the present invention are clear, thus, as the relative advantages are clear.

Further variants of the embodiment described above are possible, without departing from the teaching of the invention. Finally, it is clear that the system for monitoring the expiry date of drugs or of first aid or emergency kits thus designed is subject of numerous modifications and variations; furthermore, all the details can be replaced by technically equivalent elements. Basicallly, the materials used, as well as the dimensions, may be any according to the technical requirements.

## Claims

1. System (100) for monitoring the expiry date of a drug or a first aid or emergency kit comprising:
- at least one intelligent portable device (50),
- a drug packaging (20) containing a drug or a first aid or emergency box, bag or kit (30) containing drugs and/or medication devices, wherein said packaging (20) or box, bag or kit (30) comprises at least one wireless communication module (40) configured to communicate with a corresponding wireless communication module (51) of an intelligent portable device (50).

2. Sistem (100) according to claim 1, wherein the wireless communication module (40) is placed on the outer frontal surface of the drug packaging (20) or to the first aid or emergency box, bag or kit (30).

3. Sistem (100) according to any one of claims from 1 to 2, wherein said system comprises an identification code (Idc; Idcon), said wireless communication module (40) being configured to transmit to the intelligent portable device (50) said identification code (Idc; Idcon).

4. Sistem (100) according to any one of claims from 1 to 3, wherein the wireless communication module (40) comprises a memory unit (41), and wherein the wireless communication module (51) of the intelligent portable device (50) being configured to transmit to the wireless communication module (40) name (namprod) and expiry date (expprod) of a specific drug or kit, wherein said name (namprod) and expiry date (expprod) were previously entered into the intelligent portable device (50) by means of an application (52) of the intelligent portable device (50), the name (namprod) and the expiry date (expprod) being stored in said memory unit (41).

5. Sistem (100) according to any one of claims from 1 to 4, further comprising visual signalling means (70) and a timer (42) in electrical communication with said visual signalling means (70), said timer (42) being configured to perform a countdown between a current date (datecurr) and the expiry date of the drug or kit (expprod) and activate lighting-up on of the visual signalling means (70).

6. Sistem (100) according to claim 5, wherein said visual signalling means (70) comprise an array of LEDs (71) and wherein said timer (42) is configured to activate the lighting-up of a number of LEDs (71) equal to the difference between the expiy date of the drug or kit (expprod) and the current date (datecurr).

7. Sistem (100) according to any one of claims from 1 to to 6, wherein:
- the wireless communication module (40) comprises an embossing of an alphanumeric or Braille character (72) on its outer surface opposite to that of the drug packaging (20) or of the first aid or emergency box, bag or kit (30) and
- the intelligent portable device (50) further comprises sound communication means (53).

8. Sistem (100) according to claim 7, wherein the alphanumeric or Braille character (72) is an alphabetic letter, which is the initial of the colour of the wireless communication module (40).

9. Method for monitoring the expiry date of a drug or a first aid or emergency kit by the sistem according to any one of claims from 1 to to 8 comprising the following step:
a) providing a drug packaging (20) containing a drug or a first aid or emergency box, bag or kit (30) containing drugs and/or medication devices, wherein said packaging (20) or box (30) compraises at least one wireless communication module (40),
b) by means of the application (52) of the intelligent portable device (50), entering the name (namprod) and expiry date (expprod) of a specific drug or first aid or emergency kit,
c) the intelligent portable device (50) transmits the data of step b) to the wireless communication module (40) which stores them in the memory unit (41),
d) the wireless communication module (40) by means of visual signalling means (70) and a timer (42), displays the residual duration time of the drug or of the first aid or emergency kit; or, alternatively, upon activation of the application (52) of the intelligent portable device (50), the intelligent portable device (50) warns the user of the approaching expiration of the drug or the first aid or emergency kit.

10. Method for visually impaired users for monitoring the expiry date of a drug or a first aid or emergency kit by the system according to any one of claims from 1 to 8, comprising the following steps:
a1) providing a drug packaging (20) containing a drug or a first aid or emergency box, bag or kit (30) containing drugs and/or medication devices, wherein said packaging (20) or box, bag or kit (30) comprises at least one wireless communication module (40), wherein said wireless communication module (40) comprises an embossing of an alphanumeric or Braille character (72) on its outer surface opposite to that of the drug packaging (20) or of a first aid or emergency box bag or kit (30),
b1) by means of the application (52) of the intelligent portable device (50), entering the name (namprod) and expiry date (expprod) of a specific drug or first aid or emergency kit,
c1) the intelligent portable device (50) transmits the data of step b1) to the wireless communication module (40) comprising an embossing of an alphanumeric or Braille character (72) which stores said data in the memory unit (41),
d1) optionally, the wireless communication module (40) by means of visual signalling means (70) and a timer (42), displays the residual duration time of the drug or the first aid or emergency kit,
e1) by means of the activation of the application (52) of the intelligent portable device (50), the intelligent portable device (50) by means of sound communication means (53) warns the visually impaired user of the approaching expiration of the drug or the first aid or emergency kit, moreover specifying the alphanumeric or Braille character of the wireless communication module (40),
f1) the user, through finger contact on the embossing of the alphanumeric or Braille character (72) of the communication module (40), recognizes the drug or the first aid or emergency kit close to the expiration.

11. Method according to claim 10, wherein in step a1) the embossing of a alphanumeric or Braille character (72) is one letter, which is the initial of the colour of the wireless communication module (40).

12. The use of a wireless communication module (40) of the portable type (60) configured to communicate with a corresponding wireless communication module (51) of an intelligent portable device (50) to monitor the expiry date of a drug or a first aid or emergency kit.
